# EUROPÄISCHE PATENTANMELDUNG

(11) **EP 0 653 217 A1**
(43) Veröffentlichungstag der Anmeldung: **17.05.1995**
(21) Anmeldenummer: 94113005.6
(22) Anmeldetag: 20.08.1994
(51) Int. Cl.: A61M 5/14, A61M 5/162

(54) **Gerät zur medizinischen Infusion oder Transfusion**

(30) Priorität: 13.11.1993 DE 9317423 U
(71) Anmelder: CLINICO INFUSIONSTECHNIK GmbH, D-36251 Bad Hersfeld (DE)
(72) Erfinder: Heinzerling, Walter Chr., D-36251 Bad Hersfeld (DE)
(74) Vertreter: von Raffay, Vincenz, Dipl.-Ing.

(57) **Zusammenfassung**

Das Gerät zur medizinischen Infusion oder Transfusion besteht aus einer Tropfkammer (9) mit Einstechteil (2), Filter (7) und Belüftungsteil (4) mit Luftfilter (5) sowie aus einem Schlauch (15) mit einem Durchflußregler (16) zur Verbindung des Tropfkammerauslaufs mit einem Anschlußstück (22) an seinem anderen Ende. Dieses Gerät soll so gestaltet werden, daß eine Mehrfachanwendung in der Größenordnung von 100 Stunden möglich ist. Dieses geschieht durch folgende Merkmale:
1. am Tropfkammerauslauf ist ein Ventilteil (8) zum Verschliessen des Tropfkammerauslaufs bei Leerlauf vorgesehen;
2. der Einstechteil (2) ist mit einer scharfen, glatten Spitze zum mehrfachen Durchstechen entsprechender Verschlußstopfen versehen;
3. der Luftfilter (5) des Belüftungsteils (4) ist durch ein Belüftungsventil (6) gegen Flüssigkeitsbenetzung geschützt;
4. im Bereich des Anschlußstücks ist eine Rückstromsperre (23) vorgesehen.

## Beschreibung

Die Erfindung betrifft ein Gerät zur medizinischen Infusion oder Transfusion nach dem Oberbegriff des Anspruches 1.

Derartige Geräte sind genormt und zwar nach DIN 58362 (Infusionsgeräte) und nach DIN 58360 (Transfusionsgeräte).

Sämtliche bekannten Geräte, auch unter Berücksichtigung der genannten Normen, sind zur einmaligen Verwendung bestimmt. Dieses geschieht insbesondere zur Sicherheit der Patienten. Eingestuft werden derartige Geräte zur einmaligen Verwendung als Arzneimittel. Ein entsprechender Aufdruck ist auf der Verpackung vorgeschrieben.

Die wesentlichen Einzelteile der Geräte werden aus den unterschiedlichsten Kunststoffen hergestellt und stellen daher eine Recourcen-Belastung dar. Darüberhinaus entsteht kontaminierter Abfall, der umweltschonend entsorgt werden muss.

Der Erfindung liegt daher die Aufgabe zugrunde, ein Gerät der eingangs genannten Art zu schaffen, das unter Einhaltung aller hygienisch vorgeschriebener Regularien sowie Anwendungsfunktionen so gestaltet ist, daß eine Mehrfachanwendung in der Grössenordnung von 100 Stunden möglich ist.

Diese Aufgabe wird durch das Kennzeichen des Anspruches 1 gelöst, wobei vorteilhafte Ausgestaltungen Gegenstand der Ansprüche 2 und 3 sind.

Durch die Mehrfachverwendung des Gerätes werden die Recourcen geschont und der kontaminierte Abfall sowie der Verpackungsaufwand werden um 40 bis 75% reduziert. Entsprechend verringern sich Lagerhaltungskosten beim Hersteller und Anwender.

Im einzelnen wird die Wirtschaftlichkeit verbessert. Der in besonderer Weise gestaltete Einstechdorn ermöglicht ein mehrfaches Einstechen auch in problematische Stopfen ohne Deformation der Schnittfläche am Dorn. Kunststoff bzw. die Kombination aus Edelstahl und Kunststoff in diesem Bereich bedingen ein neutrales Verhalten gegenüber Medikamenten. Der spezielle Benetzungsschutz im Bereich des Belüftungsventiles verhindert eine Befeuchtung des Luftfilters und damit eine mögliche Fehlfunktion bei langer Benutzung und bei Überdrücken in Infusionsflaschen. Entsprechendes gilt bei Verwendung von semiflexiblen Infusionsbehältnissen.

Durch die vergrösserte Filterfläche (Anspruch 6) wird der Langzeiteinsatz ohne Gefahr eines verminderten Durchflusses durch Partikelabscheidung gewährleistet.

Eine mögliche Luftembolie wird durch selbsttätiges Verschliessen des Ventils am Tropfkammerauslauf bei Leerlaufen der Tropfkammer verhindert, d.h. der Flüssigkeitsspiegel sinkt nie unter Tropfkammerniveau. Sicherheit besteht auch bei Parallelinfusion, bedingt durch die Rückstromsperre im Anschlussstück.

Im folgenden wird die Erfindung unter Hinweis auf die Zeichnung anhand verschiedener Ausführungsbeispiele näher erläutert.

Es zeigt:
- Fig. 1: eine teilweise geschnittene Ansicht einer Ausführungsform eines Gerätes nach der Erfindung;
- Fig. 2: eine Teilansicht einer abgewandelten Ausführungsform des Einstechteils;
- Fig. 3: eine Einzelheit einer abgewandelten Ausführungsform des Belüftungsventils;
- Fig. 4: eine Teilansicht des Unterteils der Tropfkammer einer anderen Ausführungsform des Ventilteils in diesem Bereich; und
- Fig. 5: eine Ansicht einer Zuspritzmöglichkeit mit Hilfe einer Y-Abzweigung.

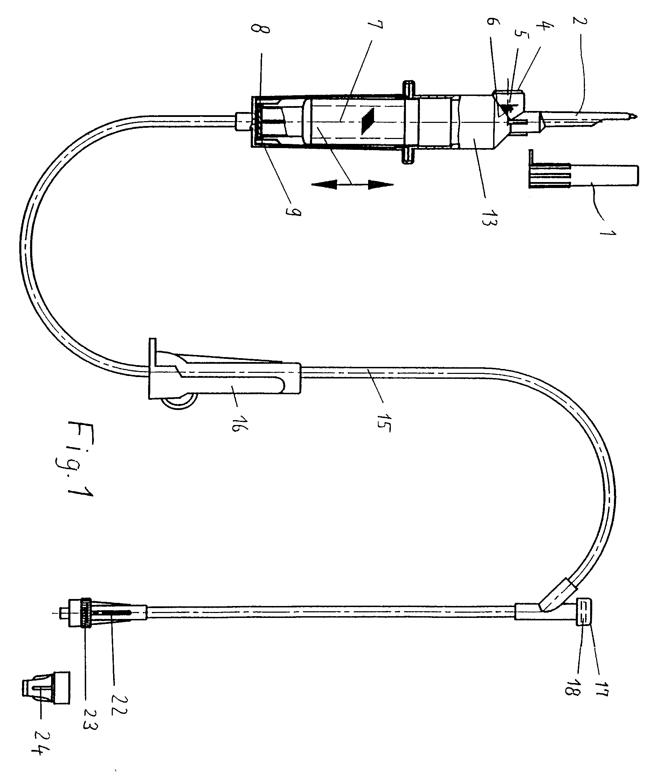

In Fig. 1 ist eine Ausführungsform eines Gerätes zur medizinischen Infusion oder Transfusion dargestellt. Dieses besteht aus einer Tropfkammer (Unterteil 9 und Oberteil 13) mit einem Einstechdorn 2, der durch eine Schutzkappe 1 verschliessbar ist.

Am Oberteil 13 ist ein Belüftungsstopfen 4 mit Belüftungsfilter 5 vorgesehen. Der Belüftungsfilter 5 wird durch ein Belüftungsventil 6 gegen Flüssigkeitsbenetzung geschützt.

In der Tropfkammer ist ein Schwimmfilter 7 auf- und abbewegbar, an dessen Boden eine Dichtscheibe 8 vorgesehen ist, die praktisch ein Ventilteil bildet. Im Bereich des Tropfkammerauslaufs ist ein entsprechender Ventilsitz vorhanden.

Dieser axial verschiebbare Schwimmfilter 7 verhindert im Falle eines Leerlaufens des Infusionsbehältnisses durch selbsttätiges Schliessen des Tropfkammerauslauf ein Leerlaufen und schützt damit vor Luftembolie.

Wenn es sich um ein Infusionsgerät handelt, liegt die Maschenweite der vergrösserten Filterfläche des Schwimmfilters 7 im Bereich von 1 - 20 µm. Bei einem Transfusionsgerät liegt die Maschenbreite bei ca. 200µm.

Vom Tropfkammerauslauf führt ein Schlauch 15 mit einem Druchflussregler (Schlauchklemme 16) über ein Y-Abzweigstück 17 mit Zuspritzscheibe 18 zu einem Konektor oder Anschlußstück 22 mit Rückstromsperre 23. Eine Verschlusskappe ist mit 24 bezeichnet. Es ist auch möglich, zwei oder mehrere Rückstromsperren hintereinander anzuordnen. Die Rückstromsperre verhindert bei einer Parallelinfusion einen Rückfluss in bereits leergelaufene Infusionssysteme.

Der Einstechdorn 2 der Ausführungsform nach Fig. 1 weist eine Stahlspitze auf. Der Einstechdorn 3 nach Fig. 2 ist aus einem besonderen Kunststoff hergestellt, der eine scharfe Spitze und eine mikroglatte Oberfläche gewährleistet. Hierdurch ist ein mehrfaches Durchstechen entsprechender Verschlussstopfen sichergestellt.

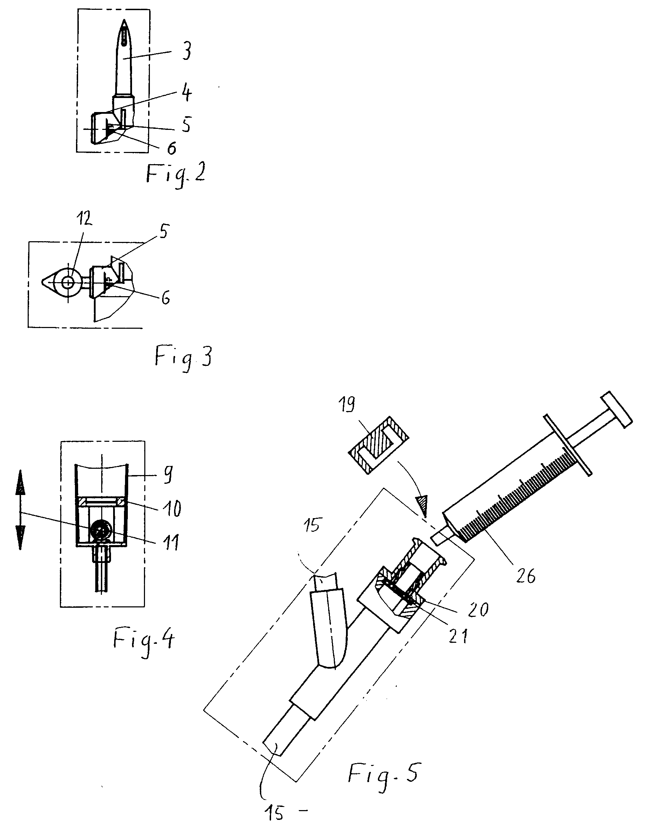

In Fig. 3 ist ein etwas anders gestaltetes Belüftungsteil dargestellt. Anstelle des Belüftungsstopfens 4 (Fig. 1 und 2) ist eine Belüftungsklappe 12 vorgesehen.

Bei der Ausführungsform nach Fig. 4 ist der Filter ein einfacher Flüssigkeitsfilter 10. Das Ventil im Bereich des Tropfkammerauslaufs wird durch eine Kugel 11 gebildet, die mit einem entsprechenden Ventilsitz am Auslauf zusammenarbeitet.

In Fig. 5 ist eine andere Ausführungsform einer Y-Abzweigung 20 mit Zuspritzventil 21 dargestellt. Bei dieser Ausführungsform kann ohne Injektionsnadel lediglich durch eine Standardspritze 26 in den Luerkegel zugespritzt werden.

## Patentansprüche

1. Gerät zur medizinischen Infusion oder Transfusion, bestehend aus einer Tropfkammer (9,13) mit Einstechteil (2,3), Filter (7) und Belüftungsteil (4) mit Luftfilter (5) sowie aus einem Schlauch (15) mit einem Durchflussregler (16) zur Verbindung des Tropfkammerauslaufs mit einem Anschlusstück (22) an seinem anderen Ende, gekennzeichnet durch die Ausbildung zur Mehrfachverwendung durch folgende Merkmale:
1. am Tropfkammerauslauf ist ein Ventilteil (8,11) zum Verschliessen des Tropfkammerauslaufs bei Leerlauf vorgesehen;
2. der Einstechteil (2,3) ist mit einer scharfen, glatten Spitze zum mehrfachen Durchstechen entsprechender Verschlussstopfen versehen;
3. der Luftfilter (5) des Belüftungsteils (4) ist durch ein Belüftungsventil (6) gegen Flüssigkeitsbenetzung geschützt;
4. im Bereich des Anschlussstücks ist eine Rückstromsperre (23) vorgesehen.

2. Gerät nach Anspruch 1, dadurch gekennzeichnet, daß der Filter als Schwimmfilter mit vergrösserter Filteroberfläche ausgebildet ist und als Ventilteil eine Dichtscheibe (8) trägt, die dem Tropfkammerauslauf gegenüberliegt.

3. Gerät nach Anspruch 1, dadurch gekennzeichnet, daß das Ventilteil als Kugel (11) ausgebildet ist, die bei Leerlauf der Tropfkammer auf einem den Tropfkammerauslauf umgebenden Ventilsitz zur Anlage gelangt.

4. Gerät nach einem der vorstehenden Ansprüche 1 bis 3, dadurch gekennzeichnet, daß das Einstechteil (3) ein scharfe, mikroglatte Kunststoffspitze aufweist (Fig. 2).

5. Gerät nach einem oder mehreren der vorstehenden Ansprüche 1 bis 3, dadurch gekennzeichnet, daß das Einstechteil (2) eine in Kunststoff integrierte Stahlspitze aufweist (Fig. 1).

6. Gerät nach Anspruch 2, dadurch gekennzeichnet, daß die Filterfläche des Schwimmfilters (7) für Transfusionsgeräte eine Maschenweite in der Grössenordnung von ca. 200 µm und für Infusionsgeräte von 1 - 20 µm aufweist.

7. Gerät nach einem oder mehreren der vorstehenden Ansprüche, dadurch gekennzeichnet, daß die äussere Abdeckung des Belüftungsventils (4) einen starren Belüftungsstopfen aufweist.

8. Gerät nach einem der vorstehenden Ansprüche 1 bis 6, dadurch gekennzeichnet, daß die äussere Abdeckung des Belüftungsventils mit einer verschliessbaren Belüftungsklappe (12) versehen ist (Fig. 3).

9. Gerät nach einem oder mehreren der vorstehenden Ansprüche, dadurch gekennzeichnet, daß der Schlauch (15) eine Abzweigung (17) mit integrierter Zuspritzscheibe (18) oder mit Zuspritzventilen (21) aufweist.
